# EUROPEAN PATENT SPECIFICATION

(11) **EP 3 527 378 B1**
(45) Date of publication and mention of the grant of the patent: **23.09.2020**
(21) Application number: 17860136.5
(22) Date of filing: 29.09.2017
(51) Int. Cl.: B41J 2/14, B41J 2/01

(54) **LIQUID DISCHARGE DEVICE, INSPECTION DEVICE HAVING LIQUID DISCHARGE DEVICE, AND CELL CULTURE DEVICE HAVING LIQUID DISCHARGE DEVICE**
FLÜSSIGKEITSAUSSTOSSVORRICHTUNG, INSPEKTIONSVORRICHTUNG MIT FLÜSSIGKEITSAUSSTOSSVORRICHTUNG UND ZELLKULTURVORRICHTUNG MIT FLÜSSIGKEITSAUSSTOSSVORRICHTUNG
DISPOSITIF DE DÉCHARGE DE LIQUIDE, DISPOSITIF D'INSPECTION POURVU D'UN DISPOSITIF DE DÉCHARGE DE LIQUIDE, ET DISPOSITIF DE CULTURE DE CELLULES POURVU D'UN DISPOSITIF DE DÉCHARGE DE LIQUIDE

(30) Priority: 13.10.2016 JP 2016201714
(43) Date of publication of application: 21.08.2019
(73) Proprietor: Panasonic Intellectual Property Management Co., Ltd., Osaka-shi, Osaka 540-6207 (JP)
(72) Inventor: YAMAMOTO Kenichi, Osaka 540-6207 (JP); NAKAGAWA Tohru, Osaka 540-6207 (JP); YOSHIDA Hidehiro, Osaka 540-6207 (JP)
(74) Representative: Vigand, Philippe
(86) International application number: PCT/JP2017/035377
(87) International publication number: WO 2018/070264

(56) References cited:
- WO-A1-02/070133
- JP-A- H06 344 549
- JP-A- 2000 137 034
- JP-A- 2001 232 245
- JP-A- 2004 239 844
- JP-A- 2005 081 643
- JP-A- 2007 209 888
- JP-A- 2008 114 569
- US-A1- 2002 150 511
- US-A1- 2013 206 857
- US-A1- 2016 288 117

## Description

### TECHNICAL FIELD

The technical field relates to a liquid discharge device for discharging liquid, an inspection device having the liquid discharge device, and a cell culture device having the liquid discharge device. Particularly, the technical field relates to a liquid discharge device suited for supplying or dispensing a liquid, such as a chemical and a reagent, in a range of fields including drug discovery, medicine, and biotechnology, and to an inspection device having such a liquid discharge device, and a cell culture device having the liquid discharge device.

### BACKGROUND

An inkjet printing device has gained popularity in the last years, and is widely used as a device for recording texts and images on various types of recording media. The applicable areas of inkjet technology are not limited to printing on paper and films, and the inkjet technology has been used in the fields of drug discovery, medicine, and biotechnology. Indeed, the inkjet technology has a wide range of applications.

Specifically, there have been attempts to supply trace amounts of chemicals and reagents to analytes or the like by taking advantage of the accurate discharge performance of the inkjet technology.

However, analytes used in the fields of drug discovery, medicine, and biotechnology are sensitive to metal ions, and portions that come into contact with the liquid need to be metal-free. In changing chemicals and reagents, mixing with new chemicals and reagents also needs to be avoided because it causes a performance drop, and produces an unexpected effect due to their interaction.

However, the inside of a common liquid discharge head is configured from metallic material, and includes narrow fluid channels and spaces. It is accordingly not possible to thoroughly wash inside of a liquid discharge head. To overcome this shortcoming, a liquid discharge head is proposed that includes an easily replaceable liquid contact unit (JP-A-2008-114569).

The liquid discharge head proposed by this related art includes a detachable container, and the container is pressurized from outside using a drive mechanism. The liquid inside the container can discharge through an open-end nozzle as the pressure inside the container increases with increase in the displacement of the drive mechanism.

However, while the container pressure can directly increase under applied pressure, the container is not able to follow a reduced pressure, or a reducing displacement of the drive mechanism. This is because the pressure change is controlled by the resiliency of the container, and the container cannot quickly return to its initial position. This prevents discharge at high repetition frequency.

Finally, document US 2016288117 A1 discloses liquid droplet dispenser, and document WO 20070133 A1 discloses a piezoelectric pipetting device housing and methods for making and using the same.

### SUMMARY

The present disclosure has been made to provide a solution to the foregoing problem, and it is an object of the present disclosure to provide a liquid discharge device that, despite using a disposable pressure chamber, can repeatedly operate at high speed. The present disclosure is also intended to provide an inspection device having such a liquid discharge device, and a cell culture device having the liquid discharge device.

According to an aspect of the disclosure, a liquid discharge device is provided according to claim 1.

According to another aspect of the disclosure, an inspection device is used that includes the liquid discharge device.

According to yet another aspect of the disclosure, a cell culture device is used that includes the liquid discharge device.

With the aspect of the disclosure, the displacement created by the drive part can be reliably transmitted to the nozzle, and can actively act in both compression and expansion directions of the nozzle.

By allowing the nozzle to more accurately follow the displacement created by the drive part, the nozzle can discharge liquid at high repetition frequencies.

The drive part, and the holding part of the nozzle can remain in contact with each other regardless of the repetition frequency, and the wear caused by impact on the contact surfaces of the drive part and the nozzle when these members are repeatedly brought into contact with each other can be prevented to achieve a longer nozzle life. This can reduce wasted nozzles.

### BRIEF DESCRIPTION OF THE DRAWINGS

FIG. 1A is a perspective view showing a structure of a liquid discharge device of First Embodiment.
FIG. 1B is a cross sectional view showing a structure of the liquid discharge device of First Embodiment.
FIG. 1C is a perspective view showing a discharge portion of the liquid discharge device of First Embodiment.
FIG. 2 is a graph representing a relationship between the amount of discharged liquid droplet per unit time, and discharge frequency in the liquid discharge device of First Embodiment.
FIG. 3 is a graph representing a relationship between the amount of discharged liquid droplet, and the number of discharges in the liquid discharge device of First Embodiment.
FIG. 4A shows an elevational view and a top view representing a variation of the nozzle of First Embodiment.
FIG. 4B shows an elevational view representing a variation of the nozzle of First Embodiment.
FIG. 5 is a diagram representing a structure of a liquid discharge device of Second Embodiment.
FIG. 6A is a side view of a liquid discharge device of Third Embodiment.
FIG. 6B is an elevational view of a test unit of Third Embodiment.
FIG. 7A is a side view of a cell culture device of Third Embodiment.
FIG. 7B is a plan view of a container of Third Embodiment.

### DESCRIPTION OF EMBODIMENTS

Embodiments of the present disclosure are described below, with reference to the accompanying drawings.

### First Embodiment

### Configuration

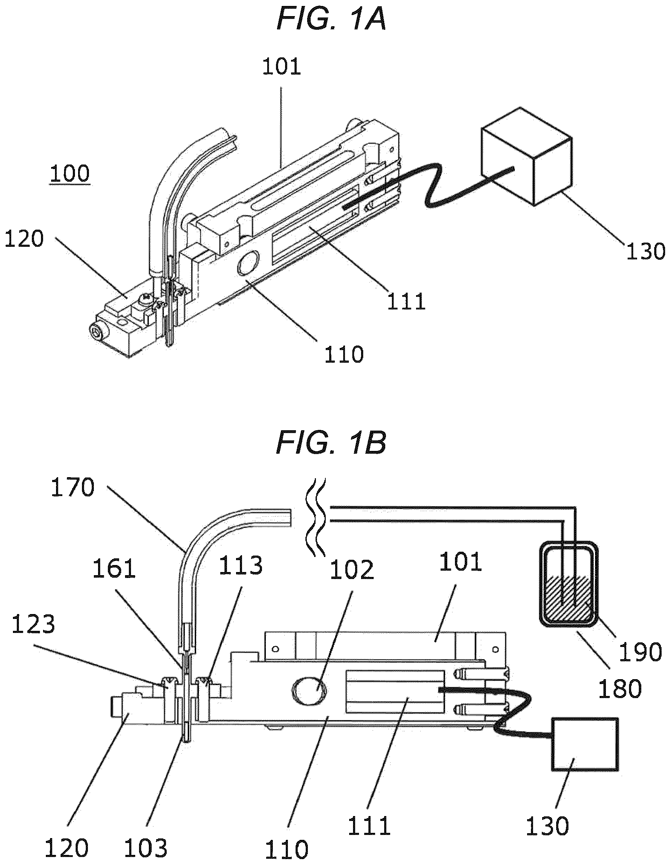

FIG. 1A is a schematic view showing an inner structure of a liquid discharge device of First Embodiment of the present disclosure as viewed from an oblique direction. The liquid discharge device 100 includes a drive part 110 and a fixing part 120 installed in a base 101. The drive part 110 includes a piezoelectric element 111 that creates displacement. The piezoelectric element 111 creates displacement as it expands and contracts in synchronism with a drive pulse signal generated by an external waveform generator 130.

FIG. 1B is a schematic view showing an inner structure of the liquid discharge device 100 of First Embodiment of the present disclosure as viewed from the side. The drive part 110 is fixed at the rear of the base 101. The drive part 110 is held in place with a fixing rod 102. The drive part 110, to which the fixing rod 102 is inserted, has an ellipsoidal hole formed therein with a major axis extending in a direction of displacement of the piezoelectric element 111 so that the displacement of the piezoelectric element 111 is transmitted forward.

A drive pin 113 is provided at an end portion of the drive part 110. The drive pin 113 moves with the drive part 110.

A fixing pin 123 having the same shape as the drive pin 113 is provided at an end portion of the fixing part 120. The fixing pin 123 is held by the fixing part 120, and does not move.

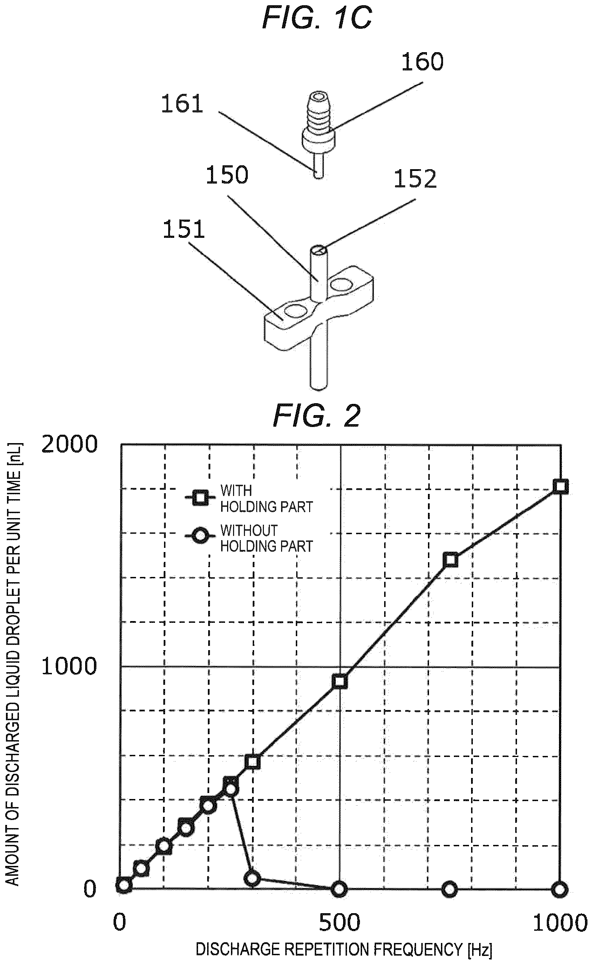

An insertion hole 103 is provided between the drive pin 113 and the fixing pin 123, and a nozzle 150 is inserted in the insertion hole 103. FIG. 1C is a perspective view of the nozzle 150 of First Embodiment of the present disclosure . The nozzle 150 is provided with a pair of radial holding parts 151 (a first holding part and a second holding part) in a 180-degree position. The holding parts 151 each have a fitting hole that is slightly smaller in diameter than the drive pin 113 and the fixing pin 123. With the drive pin 113 and the fixing pin 123 fitted into the fitting holes, the horizontal and vertical positions of the nozzle 150 can be specified with good repeatability.

The drive pin 113 and the fixing pin 123 have increasing diameters toward the tip. This is to prevent the holding parts 151 of the nozzle 150 from coming off the drive pin 113 and the fixing pin 123, and causing the nozzle 150 to fall off as a result of displacement of the drive pin 113 of the drive part 110 due to the piezoelectric element 111.

A throttle 160 is inserted in an opening 152 provided in an upper part of the nozzle 150. The throttle 160 is hollow inside. The throttle 160 has a small orifice in a portion inserted into the nozzle 150. The opposite end of the throttle 160 is knurled in a way to appear like a bamboo shoot, allowing a tube 170 to be inserted thereon and fixed. The tube 170 connects to a tank 180.

### Supply of Chemicals

By pressurizing the tank 180, a chemical 190 can be forced into the tube 170 from the tank 180, and the throttle 160 and the nozzle 150 are filled with the chemical 190 through the tube 170. With the liquid discharge device 100 fully charged with the chemical, preparation for discharge is complete.

### Discharge Operation

In response to a discharge signal sent by the waveform generator 130 to drive the piezoelectric element 111, the piezoelectric element 111 undergoes a displacement that varies with the waveform of the discharge signal. Here, when the discharge signal has a positive voltage, the piezoelectric element 111 is displaced to expand. The displacement is transmitted to the drive pin 113 via the drive part 110, and acts to compress the nozzle 150 from the side. Here, the tip portion of the nozzle 150, and the orifice 161 of the throttle 160 create a pseudo closed space of large channel resistance, and this space acts as a pressure chamber for increasing the internal pressure.

Accordingly, the compressive displacement acting sideways on the nozzle 150 causes the chemical 190 to discharge from the tip portion of the nozzle 150 as a result of the pressure increase in the pressure chamber.

After a lapse of a predetermined time period, the output of the waveform generator 130 starts to decrease. As the applied voltage to the piezoelectric element 111 decreases, the piezoelectric element 111 returns to its original state from the expanded state. That is, a transition occurs from expansion to contraction. In the nozzle 150, this transition corresponds to a change from compression to expansion.

As a rule, the time from compression to expansion upon release of compressive pressure is governed by the elastic modulus. Polypropylene - a resin material used for the nozzle 150 of the present embodiment - has an elastic modulus of 1.12 to 1.58 GPa, and, experimentally, the nozzle 150 requires about 50 milliseconds to complete the voluntary expansion. The piezoelectric element 111, on the other hand, is responsive to voltage. The response time of the piezoelectric element 111 is only about 50 microseconds, much shorter than the voluntary recovery time of polypropylene, though it is restricted by shape.

Because the holding parts 151 of the nozzle 150 are restricted by the drive pin 113 and the fixing pin 123, the expansion time of the nozzle 150 can be more actively controlled. That is, the expansion time of the nozzle 150 can be brought closer to the contraction time of the piezoelectric element 111.

This means that compression and expansion of the nozzle 150 can take place at a high repetition frequency. In other words, a high-frequency discharge is possible.

Preferably, the drive pin 113 and the fixing pin 123 are disposed opposite each other with respect to the nozzle 150. Preferably, the drive pin 113 and the fixing pin 123 are disposed in point symmetry about the nozzle 150.

FIG. 2 represents a relationship between an amount of discharged liquid droplet per unit time, and discharge repetition frequency. In the absence of the holding parts 151, the amount of discharged liquid droplet per second with respect to the repetition frequency (discharge repetition frequency) becomes less linear after about 200 Hz. After peaking at about 250 Hz, the discharge amount rapidly decreases before the liquid completely fails to discharge.

This is a result of the nozzle 150 failing to follow the drive part 110, causing the drive part 110 to retract after the nozzle 150 is compressed, and start the next compression before the nozzle 150 expands. With the nozzle 150 being in a compressed state and unable to expand, the pressure inside the nozzle 150 cannot be increased, and the liquid cannot discharge.

With the holding parts 151 provided for the nozzle 150 in the liquid discharge device 100 of the present embodiment, the discharge quality can be maintained even at 1,000 Hz, though changes in the amount of discharged liquid droplet are observed.

With the drive pin 113 and the fixing pin 123 fixing the holding parts 151 of the nozzle 150, it is possible to permanently maintain contact even at high repetition frequencies. This increases the life of the nozzle 150.

With no such contact, the expansion of the nozzle 150 cannot follow the drive pin 113 retracting in response to contraction of the piezoelectric element 111, with the result that contact cannot be maintained.

This means that the drive pin 113 collides with the nozzle 150 upon reexpansion of the piezoelectric element 111. Such a collision causes wear in the nozzle 150, and the compression distance decreases. The resulting pressure decrease becomes a large obstacle for stable discharge, and the nozzle 150 has a shorter lifetime.

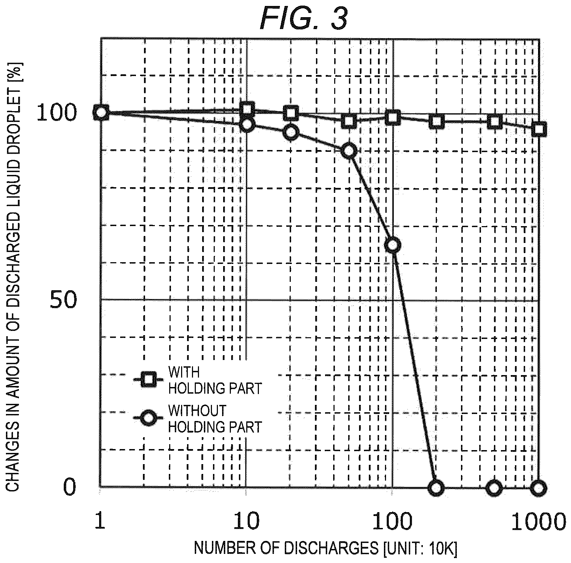

FIG. 3 is a graph representing a relationship between an amount of discharged liquid droplet, and the number of discharges. In the absence of the holding parts 151, in which case the drive part 110 and the nozzle 150 are brought into contact each other in a repeated fashion, the amount of discharged liquid droplet gradually decreases from the start, and shows an about 10% decrease from the initial value after 500,000 discharges.

The reduced amount of discharged liquid droplet is a result of the drive part 110 failing to reliably transmit its displacement to the inside of the nozzle 150 because of the wear occurring slowly but progressively in the nozzle 150 as a result of the repeated contact between the drive part 110 and the nozzle 150. With the holding parts 151 provided for the nozzle in the liquid discharge device 100 of the present embodiment, there is no decrease of the amount of discharged liquid droplet even after 10 million discharges.

### Variations of Nozzle 150

The holding parts 151 serve to reliably transmit movement of the drive pin 113 and the fixing pin 123 to the nozzle 150. However, in applications involving frequent replacement of chemicals, easy removability of the nozzle 150 is also an important factor.

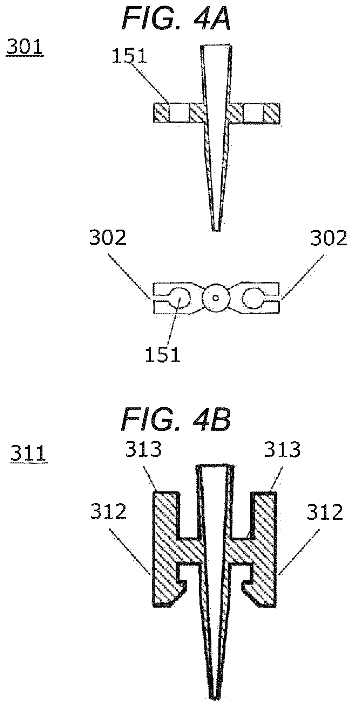

FIGS. 4A to 4B show elevational views and a top view representing variations of the nozzle 150 described in First Embodiment.

FIG. 4A shows a nozzle 301 having a cutout 302 in a portion of the holding parts 151. With the cutouts 302, the nozzle 301 can be inserted and removed with a smaller force, particularly in removing the nozzle 301.

FIG. 4B shows a side view of a nozzle 311. The nozzle 311 has hooks 312 provided along the direction of insertion of the nozzle 311 to join the nozzle 311 to the drive part 110 and the fixing part 120. The hooks 312 have deformable portions 313, which, by being flexible, make insertion and removal easier, particularly in removing the nozzle 311. When providing the hooks 312, recesses for accepting the hooks 312 need to be provided for the drive part 110 and the fixing part 120.

With the flexible deformable portions 313, the hooks 312 make insertion and removal easier, particularly in removing the nozzle, as with the case of the nozzle 311. An added advantage is that the structure is simpler, and the nozzle can be produced at lower cost.

Desirably, the nozzle has a low elastic modulus to provide deformability. The nozzle has been described as being made of a single material. However, for a longer life, the nozzle is preferably made of a high-elastic-modulus material. It is also effective to form the nozzle from different materials, either by molding the nozzle in two steps using different materials, or bonding different materials after separately forming these materials.

### Second Embodiment

### Configuration

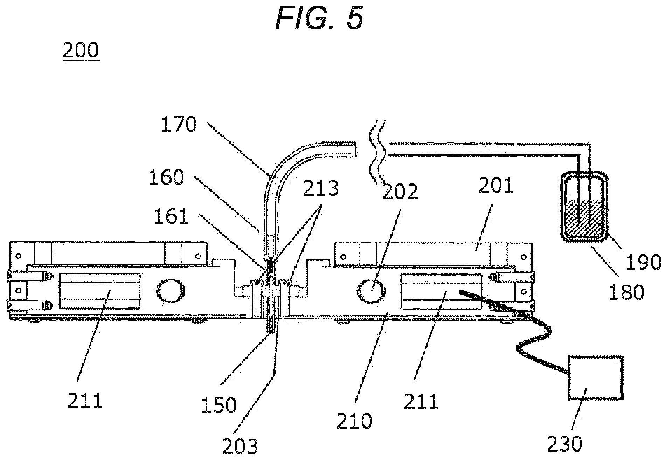

FIG. 5 is a schematic view showing a cross sectional structure of a liquid discharge device 200 of Second Embodiment as viewed from the side. Anything that is not described is the same as in First Embodiment. Second Embodiment differs from First Embodiment in that the nozzle is pressurized and compressed from both sides.

The liquid discharge device 200 includes a pair of drive parts 210 installed in a base 201. The drive parts 210 include piezoelectric elements 211 that create displacement. The piezoelectric elements 211 create displacement as they expand and contract in synchronism with a drive pulse signal generated by an external waveform generator 230.

The drive parts 210 are fixed at the ends of the base 201. The drive parts 210 are held in place with fixing rods 202. The drive part 210, to which the fixing rod 202 is inserted, has an ellipsoidal hole formed therein with a major axis extending in a direction of displacement of the piezoelectric element 211 so that the displacement of the piezoelectric element 211 is transmitted toward the center of the base 201.

A pair of drive pins 213 is provided at end portions of the drive parts 210. Between the drive pins 213 is provided an insertion hole 203 to which the nozzle 150 is inserted.

A throttle 160 is inserted in the nozzle 150. The throttle 160 connects to a tank 180 via a tube 170.

### Supply of Chemicals

By pressurizing the tank 180, the throttle 160 and the nozzle 150 can be filled with a chemical 190 via the tube 170. With the liquid discharge device 200 fully charged with the chemical, preparation for discharge is complete.

### Discharge Operation

In response to a discharge signal sent by the waveform generator 230 to drive the piezoelectric elements 211, the piezoelectric elements 211 undergo a displacement that varies with the waveform of the discharge signal. Here, when the discharge signal has a positive voltage, the piezoelectric elements 211 are displaced to expand. The displacement is transmitted to the drive pins 213 via the drive parts 210, and acts to compress the nozzle 150 from both sides. Here, the tip portion of the nozzle 150, and the orifice 161 of the throttle 160 create a pseudo closed space of large channel resistance, and this space acts as a pressure chamber for increasing the internal pressure. Accordingly, the compressive displacement acting sideways on the nozzle 150 causes the chemical 190 to discharge from the tip portion of the nozzle 150 as a result of the pressure increase in the pressure chamber.

In the present embodiment, the pair of drive parts compresses the nozzle from both sides, and the displacement that drives the nozzle is apparently two times higher. This makes it possible to discharge a larger droplet than with the liquid discharge device 100 of First Embodiment, though the size of the liquid discharge device increases.

The liquid discharge device can be used to make devices for applying various types of liquids. The object that is moved for application of a liquid may be the object to be applied with the liquid, or may be the liquid discharge device itself. The liquid discharge device may be configured as an applicator that includes a drive part, and a controller.

### Effects

The liquid discharge devices of First and Second Embodiments include the holding parts 151 in the nozzle for discharging the liquid. This ensures that the drive part and the nozzle are in contact with each other at all times, and the operation of the drive part can be reliably transmitted to the nozzle. The liquid discharge devices, despite allowing for replacement of the liquid contact unit, can therefore discharge liquid at a high repetition frequency while achieving a long nozzle life.

### Third Embodiment

With the liquid discharge devices 100 and 200 described above, trace amounts of liquid can be accurately applied in certain quantities for extended time periods.

These devices are therefore applicable to application of blood to, for example, a biosensor or a biochip. For example, in applying blood to a blood sugar sensor, the liquid discharge device 100 or 200 is used to apply a droplet of blood to the blood sugar sensor, and the glucose level is found by reading the sensor with a detector. In this way, blood can be applied in accurate quantities, and the results are accurate accordingly.

Aside from glucose sensors, the liquid discharge device is also applicable to immunosensors such as a tumor marker, and a heart disease marker. The sensors may be DNA sensors, such as for epigenetics and infections. The sensors may be a wide range of biosensors.

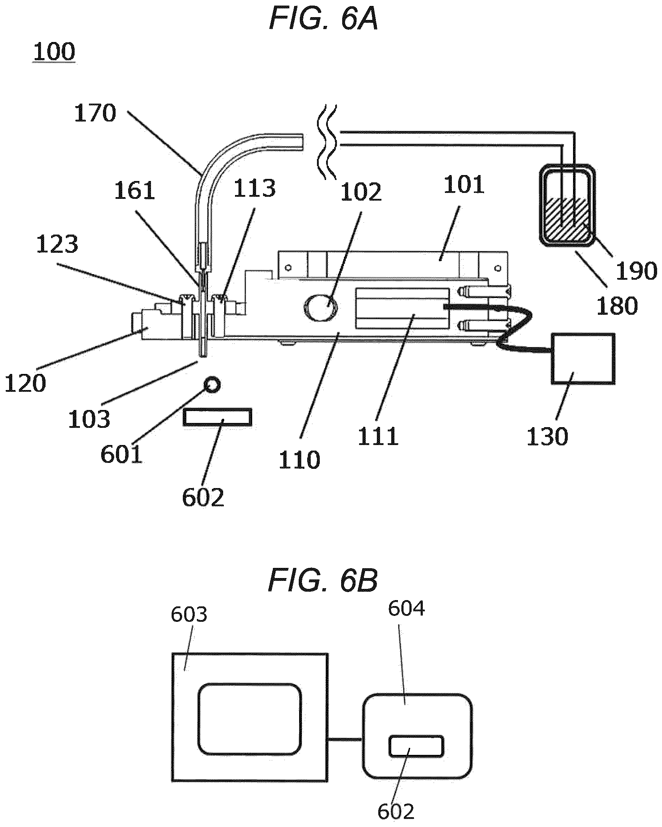

A device of Third Embodiment is described below, with reference to FIGS. 6A and 6B. Anything that is not described is the same as in the foregoing First and Second Embodiments.

FIG. 6A is a side view of a liquid discharge device 100. The liquid discharge device 100 discharges blood (liquid 601) to a sensor 602.

The sensor 602 is analyzed with the test unit shown in FIG. 6B. FIG. 6B is an elevational view of the test unit. The sensor 602 is inserted in the detector 604 shown in FIG. 6B. The detector 604 tests the sensor 602 under the control of a controller 603, and displays the result.

FIGS. 6A and 6B represent an inspection device.

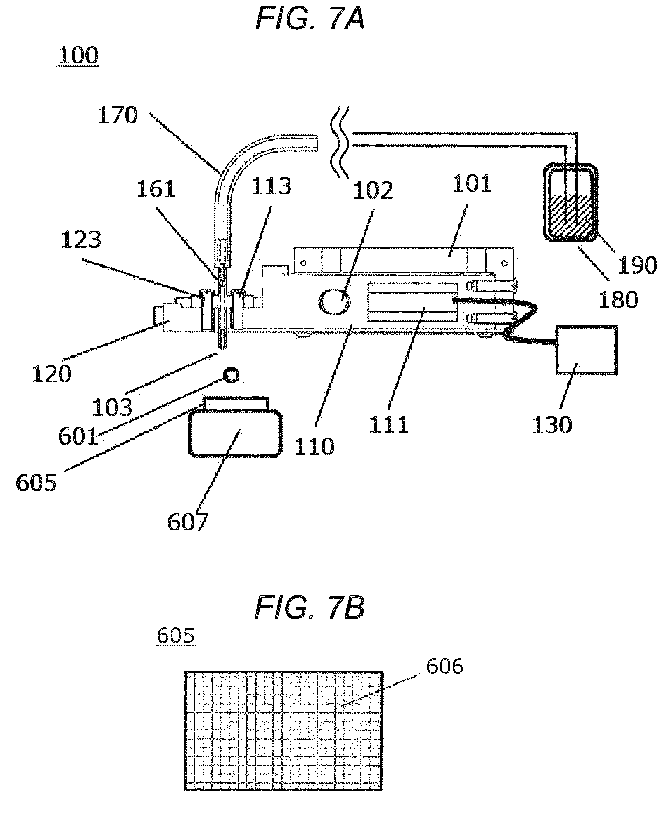

The sensor 602 may be replaced with the container 605 shown in FIG. 7B. FIGS. 7A and 7B represent an example of a cell culture device. FIG. 7B is a plan view of the container 605. FIG. 7A is a side view of the cell culture device. The container 605 has a plurality of wells 606. For use as a cell culture device, cells are retained in the wells 606, and a culture medium is applied to the wells 606 with the discharge device 100. In this case, liquid droplets need to be applied to the wells 606, and a stage 607 is provided underneath the container 605 so that the container 605 is movable within a plane.

The container 605 may have only one well 606, and a liquid droplet may discharge into the single well 606 multiple times. A cell sheet may be placed in the well 606, and the liquid 601 may be applied to the cell sheet.

In order to produce a sensor, the device may be used to apply various solutions to a sensor in the manner described above.

### Final Note

First to Third Embodiments may be combined.

The mechanism of the holding parts 151, and the mechanism of the drive pin 113 and fixing pin 123 are interchangeable. That is, the holding parts 151 may be pins, and the drive pin 113 and the fixing pin 123 may have through holes. The mechanism is not limited, as long as these can be fitted to each other.

At least one of the two holding parts (a first holding part and a second holding part) is held by the drive part. Preferably, the other holding part (a first holding part or a second holding part) is held by the fixing part or the drive part.

The liquid discharge device can preferably be used as a device for supplying or dispensing liquids such as chemicals and reagents in applications such as in drug discovery, medicine, and biotechnology. The liquid discharge device also can be used to supply or dispense liquids for experiments, mass production, and testing.

### Reference Signs List

- 100: LIQUID DISCHARGE DEVICE
- 101: BASE
- 102: FIXING ROD
- 103: INSERTION HOlE
- 110: DRIVE PART
- 111: PIEZOELECTRIC ELEMENT
- 113: DRIVE PIN
- 120: FIXING PART
- 123: FIXING PIN
- 130: EXTERNAL WAVEFORM GENERATOR
- 150: NOZZLE
- 151: HOLDING PARTS
- 152: OPENING
- 160: THROTTLE
- 161: ORIFICE
- 170: TUBE
- 180: TANK
- 190: CHEMICAL
- 200: LIQUID DISCHARGE DEVICE
- 201: BASE
- 202: FIXING ROD
- 203: INSERTION HOLE
- 210: DRIVE PARTS
- 211: PIEZOELECTRIC ELEMENT
- 213: DRIVE PIN
- 230: EXTERNAL WAVEFORM GENERATOR
- 301: NOZZLE
- 311: NOZZLE
- 312: HOOKS
- 313: DEFORMABLE PORTIONS
- 321: NOZZLE
- 322: HOOK
- 323: DEFORMATION PART
- 601: LIQUID
- 602: SENSOR
- 603: CONTROLLER
- 604: DETECTOR
- 605: CONTAINER
- 606: WELL
- 607: STAGE

## Claims

1. A liquid discharge device (100 ; 200) comprising:
a nozzle (150 ; 301 ; 311) for discharging a liquid droplet;
a base (101 ; 201) to which the nozzle (150 ; 301 ; 311) is attached;
a fixing part (120) for fixing the nozzle (150 ; 301 ; 311) to the base (101 ; 201); and
a drive part (110 ; 210) that creates displacement,
**characterized in that**:
the nozzle (150 ; 301 ; 311) has a first holding part (151 ; 312) and a second holding part (151 ; 312), and
the drive part (110 ; 210) holds at least one of the first holding part (151 ; 312) and the second holding part (151 ; 312), so that the displacement created by the drive part is transmitted to the nozzle (150 ; 301 ; 311), so as to act in both compression and expansion directions of the nozzle.

2. The liquid discharge device (100 ; 200) according to claim 1, wherein whichever of the first holding part (151 ; 312) and the second holding part (151 ; 312) that is not held by the drive part (110 ; 210) is held by the fixing part (120).

3. The liquid discharge device (100 ; 200) according to claim 1, wherein the drive part (110 ; 210) holding at least one of the first holding part (151 ; 312) and the second holding part (151 ; 312) is different for the first holding part (151 ; 312) and the second holding part (151 ; 312).

4. The liquid discharge device (100 ; 200) according to claim 1 or 3, wherein the drive part (110 ; 210) comprises two drive parts (210), one holding the first holding part (151 ; 312), and one holding the second holding part (151 ; 312).

5. The liquid discharge device (100 ; 200) according to claim 1, wherein the nozzle (150 ; 301 ; 311) is detachably attached to the base (101 ; 201).

6. The liquid discharge device (100 ; 200) according to claim 1, wherein the first holding part (151 ; 312) and the drive part (110 ; 210) are held to each other with a fitting mechanism.

7. The liquid discharge device (100 ; 200) according to claim 1, wherein the drive part (110 ; 210) uses a stacked piezoelectric element.

8. The liquid discharge device (100 ; 200) according to claim 1, wherein the first holding part (151 ; 312) and the second holding part (151 ; 312) are disposed opposite each other with respect to the nozzle (150 ; 301 ; 311).

9. The liquid discharge device (100 ; 200) according to claim 1, wherein the nozzle (150 ; 301 ; 311) is cylindrical in shape, and the first holding part (151 ; 312) and the second holding part (151 ; 312) are disposed on a side surface of the cylindrical nozzle (150 ; 301 ; 311).

10. The liquid discharge device (100 ; 200) according to claim 1, wherein the first holding part (151 ; 312) and the second holding part (151 ; 312) are rectangular in shape.

11. The liquid discharge device (100 ; 200) according to claim 1, wherein the nozzle (150 ; 301 ; 311) deforms by being pressurized by the first holding part (151 ; 312) and the second holding part (151 ; 312).

12. An inspection device comprising:
the liquid discharge device (100 ; 200) of claim 1;
a sensor (602) that receives a liquid (601) applied by the liquid discharge device (100 ; 200);
a tester for detecting the sensor (602); and
a controller (603) for controlling the tester.

13. A cell culture device comprising:
the liquid discharge device (100 ; 200) of claim 1;
a container (605) that receives a liquid (601) applied by the liquid discharge device (100 ; 200); and
a stage (607) for moving the container.

## Patentansprüche

1. Flüssigkeitsausgabevorrichtung (100, 200), die Folgendes umfasst:
eine Düse (150; 301; 311) zum Ausgeben eines Flüssigkeitströpfchens;
eine Basis (101; 201), an der die Düse (150; 301; 311) befestigt ist;
ein Fixierteil (120) zum Fixieren der Düse (150; 301; 311) an der Basis (101; 201) und
ein Antriebsteil (110; 210), das einen Versatz schafft,
**dadurch gekennzeichnet, dass**:
die Düse (150; 301; 311) ein erstes Halteteil (151; 312) und ein zweites Halteteil (151; 312) aufweist und
das Antriebsteil (110; 210) mindestens eines des ersten Halteteils (151; 312) und des zweiten Halteteils (151; 312) derart hält, dass der Versatz, der vom Antriebsteil geschaffen wird, auf die Düse (150; 301; 311) übertragen wird, um sowohl in der Komprimierungs- als auch in der Expansionsrichtung der Düse zu wirken.

2. Flüssigkeitsausgabevorrichtung (100; 200) nach Anspruch 1, wobei dasjenige des ersten Halteteils (151; 312) und des zweiten Halteteils (151; 312), das nicht vom Antriebsteil (110; 210) gehalten wird, vom Fixierteil (120) gehalten wird.

3. Flüssigkeitsausgabevorrichtung (100; 200) nach Anspruch 1, wobei sich das Antriebsteil (110; 210), das mindestens eines des ersten Halteteils (151; 312) und des zweiten Halteteils (151; 312) hält, vom ersten Halteteil (151; 312) und vom zweiten Halteteil (151; 312) unterscheidet.

4. Flüssigkeitsausgabevorrichtung (100; 200) nach Anspruch 1 oder 3, wobei das Antriebsteil (110; 210) zwei Antriebsteile (210) umfasst, eines, das das erste Halteteil (151; 312) hält, und eines, das das zweite Halteteil (151; 312) hält.

5. Flüssigkeitsausgabevorrichtung (100; 200) nach Anspruch 1, wobei die Düse (150; 301; 311) lösbar an der Basis (101; 201) befestigt ist.

6. Flüssigkeitsausgabevorrichtung (100; 200) nach Anspruch 1, wobei das erste Halteteil (151; 312) und das Antriebsteil (110; 210) mit einem Montagemechanismus aneinander gehalten werden.

7. Flüssigkeitsausgabevorrichtung (100; 200) nach Anspruch 1, wobei das Antriebsteil (110; 210) ein gestapeltes piezoelektrisches Element verwendet.

8. Flüssigkeitsausgabevorrichtung (100; 200) nach Anspruch 1, wobei das erste Halteteil (151; 312) und das zweite Halteteil (151; 312) mit Bezug auf die Düse (150; 301; 311) einander gegenüber angeordnet sind.

9. Flüssigkeitsausgabevorrichtung (100; 200) nach Anspruch 1, wobei die Düse (150; 301; 311) von zylindrischer Form ist und das erste Halteteil (151; 312) und das zweite Halteteil (151; 312) an einer Seitenfläche der zylindrischen Düse (150; 301; 311) angeordnet sind.

10. Flüssigkeitsausgabevorrichtung (100; 200) nach Anspruch 1, wobei das erste Halteteil (151; 312) und das zweite Halteteil (151; 312) von rechteckiger Form sind.

11. Flüssigkeitsausgabevorrichtung (100; 200) nach Anspruch 1, wobei sich die Düse (150; 301; 311) verformt, indem das erste Halteteil (151; 312) und das zweite Halteteil (151; 312) Druck auf sie ausüben.

12. Prüfvorrichtung, die Folgendes umfasst:
die Flüssigkeitsausgabevorrichtung (100; 200) aus Anspruch 1;
einen Sensor (602), der eine Flüssigkeit (601) aufnimmt, die von der Flüssigkeitsausgabevorrichtung (100; 200) aufgebracht wird;
einen Tester zum Detektieren des Sensors (602) und
eine Steuerung (603) zum Steuern des Testers.

13. Zellkulturvorrichtung, die Folgendes umfasst:
die Flüssigkeitsausgabevorrichtung (100; 200) aus Anspruch 1;
einen Behälter (605), der eine Flüssigkeit (601) aufnimmt, die von der Flüssigkeitsausgabevorrichtung (100; 200) aufgebracht wird; und
ein Gestell (607) zum Bewegen des Behälters.

## Revendications

1. Dispositif de décharge de liquide (100 ; 200) comprenant :
une buse (150 ; 301 ; 311) pour décharger une goutte de liquide ;
une base (101 ; 201) à laquelle la buse (150 ; 301 ; 311) est fixée ;
une partie de fixation (120) pour fixer la buse (150 ; 301 ; 311) à la base (101 ; 201) ; et
une partie d'entraînement (110 ; 210) qui crée le déplacement,
**caractérisé en ce que** :
la buse (150 ; 301 ; 311) a une première partie de support (151 ; 312) et une seconde partie de support (151 ; 312), et
la partie d'entraînement (110 ; 210) supporte au moins l'une parmi la première partie de support (151 ; 312) et la seconde partie de support (151 ; 312), de sorte que le déplacement créé par la partie d'entraînement est transmis à la buse (150 ; 301 ; 311) afin d'agir à la fois dans les directions de compression et d'expansion de la buse.

2. Dispositif de décharge de liquide (100 ; 200) selon la revendication 1, dans lequel quelle que soit la première partie de support (151 ; 312) ou la seconde partie de support (151 ; 312) qui n'est pas supportée par la partie d'entraînement (110 ; 210), elle est supportée par la partie de fixation (120).

3. Dispositif de décharge de liquide (100 ; 200) selon la revendication 1, dans lequel la partie d'entraînement (110 ; 210) maintenant au moins l'une parmi la première partie de support (151 ; 312) et la seconde partie de support (151 ; 312) est différente de la première partie de support (151 ; 312) et la seconde partie de support (151 ; 312).

4. Dispositif de décharge de liquide (100 ; 200) selon la revendication 1 ou 3, dans lequel la partie d'entraînement (110 ; 210) comprend deux parties d'entraînement (210), l'une supportant la première partie de support (151 ; 312) et l'une supportant la seconde partie de support (151 ; 312).

5. Dispositif de décharge de liquide (100 ; 200) selon la revendication 1, dans lequel la buse (150 ; 301 ; 311) est fixée, de manière détachable, à la base (101 ; 201).

6. Dispositif de décharge de liquide (100 ; 200) selon la revendication 1, dans lequel la première partie de support (151 ; 312) et la partie d'entraînement (110 ; 210) sont supportées entre elles avec un mécanisme de raccord.

7. Dispositif de décharge de liquide (100 ; 200) selon la revendication 1, dans lequel la partie d'entraînement (110 ; 210) utilise un élément piézoélectrique empilé.

8. Dispositif de décharge de liquide (100 ; 200) selon la revendication 1, dans lequel la première partie de support (151 ; 312) et la seconde partie de support (151 ; 312) sont disposées à l'opposé l'une de l'autre par rapport à la buse (150 ; 301 ; 311).

9. Dispositif de décharge de liquide (100 ; 200) selon la revendication 1, dans lequel la buse (150 ; 301 ; 311) est de forme cylindrique, et la première partie de support (151 ; 312) et la seconde partie de support (151 ; 312) sont disposées sur une surface latérale de la buse cylindrique (150 ; 301 ; 311).

10. Dispositif de décharge de liquide (100 ; 200) selon la revendication 1, dans lequel la première partie de support (151 ; 312) et la seconde partie de support (151 ; 312) ont une forme rectangulaire.

11. Dispositif de décharge de liquide (100 ; 200) selon la revendication 1, dans lequel la buse (150 ; 301 ; 311) se déforme en étant mise sous pression par la première partie de support (151 ; 312) et la seconde partie de support (151 ; 312) .

12. Dispositif de contrôle comprenant :
le dispositif de décharge de liquide (100 ; 200) selon la revendication 1 ;
un capteur (602) qui reçoit un liquide (601) appliqué par le dispositif de décharge de liquide (100 ; 200) ;
un testeur pour détecter le capteur (602) ; et
un organe de contrôle (603) pour contrôler le testeur.

13. Dispositif de culture cellulaire comprenant :
le dispositif de décharge de liquide (100 ; 200) selon la revendication 1 ;
un récipient (605) qui reçoit un liquide (601) appliqué par le dispositif de décharge de liquide (100 ; 200) ; et
un étage (607) pour déplacer le récipient.
